# EUROPÄISCHE PATENTSCHRIFT

(11) **EP 3 060 643 B1**
(45) Veröffentlichungstag und Bekanntmachung des Hinweises auf die Patenterteilung: **04.12.2019**
(21) Anmeldenummer: 14789223.6
(22) Anmeldetag: 21.10.2014
(51) Int. Cl.: C12M 1/107, B01D 21/00, C12M 1/00

(54) **VERFAHREN UND VORRICHTUNG ZUR ABSCHEIDUNG SCHWERER BESTANDTEILE AUS HOCHVISKOSEM AUSGANGSSUBSTRAT VON BIOGASANLAGEN**
METHOD AND DEVICE FOR SEPARATING HEAVY COMPONENTS FROM A HIGH VISCOSITY STARTING SUBSTRATE OF BIOGAS PLANTS
PROCÉDÉ ET DISPOSITIF DE SÉPARATION DE COMPOSANTS LOURDS D'UN SUBSTRAT DE DÉPART À HAUTE VISCOSITÉ D'INSTALLATIONS DE BIOGAZ

(30) Priorität: 21.10.2013 DE 102013111592
(43) Veröffentlichungstag der Anmeldung: 31.08.2016
(73) Patentinhaber: Ellmann Engineering (EE) GmbH, 06636 Laucha (DE)
(72) Erfinder: ELLMANN, Reik, 06636 Laucha an der Unstrut (DE)
(74) Vertreter: K & H Bonapat Patentanwälte Koch · von Behren & Partner mbB
(86) Internationale Anmeldenummer: PCT/EP2014/072543
(87) Internationale Veröffentlichungsnummer: WO 2015/059138

(56) Entgegenhaltungen:
- WO-A1-98/24730
- DE-A1- 10 254 309
- DE-A1-102012 208 649
- US-A1- 2008 199 943

## Beschreibung

Die Erfindung betrifft ein Verfahren zur Abscheidung schwerer Bestandteile aus hochviskosem Ausgangssubstrat von Biogasanlagen gemäß Oberbegriff des Anspruchs 1. Die Erfindung betrifft des Weiteren eine Vorrichtung zur Durchführung des Verfahrens.

In einigen Substraten für Anaerobreaktoren von Biogasanlagen sind erhebliche Mengen an Sand, Split und kleinen Steinen in feiner Verteilung enthalten. Die hohe Viskosität der Ausgangssubstrate erlaubt keine effiziente Abscheidung dieser Bestandteile durch Schwerkraftverfahren. Eine Verdünnung der Substrate mittels zugeführter Medien zur Reduzierung der Viskosität scheidet aus, da hierdurch die Wirtschaftlichkeit des Anaerobprozesses erheblich gefährdet würde.

In der Folge bauen sich in Anaerobreaktoren unterschiedlich starke Sedimentschichten auf, die teilweise nach nur einem Jahr Betriebszeit mit erheblichem Aufwand mechanisch entfernt werden müssen. Dies bedingt eine Betriebsunterbrechung, hohe Kosten sowie Einnahmeausfälle für den Anlagenbetreiber. WO A 98/24730 beschreibt einen Biogasreaktor mit einer Vorkammer, um große und schwere Störstoffe abzufangen.

Der Erfindung liegt die Aufgabe zugrunde, ein Verfahren und eine Vorrichtung zu schaffen, mittels derer im Ausgangssubstrat mitgeführte schwere Bestandteile wie Sand, Split, Steine wirksam abgeschieden werden können, ohne die Effizienz der Abbauprozesse in der Biogasanlage zu gefährden.

Die Erfindung wird mit einem Verfahren gelöst wie im Anspruch 1 beschrieben. Eine Vorrichtung zur Durchführung des Verfahrens gemäß Erfindung beschreibt Anspruch 4. Weiterbildungen der Erfindung zeigen die Unteransprüche auf.

Das Verfahren gemäß der Erfindung nutzt den Effekt aus, dass im Fermenter bzw. Anaerobreaktor der Biogasanlage eine Reduzierung der Viskosität des Fermenterinhalts durch Abbauprozesse stattfindet. Bei Eintritt des frischen, hochviskosen Ausgangssubstrats in den Fermenter wird ein Verdünnungseffekt erzielt durch Vermischen dieses Ausgangssubstrats mit niedrigviskosem Fermenterinhalt. Hierdurch wird eine Sedimentation von Sand, Split, Steinen gefördert und eine wirksame Abscheidung dieser Stoffe bereits im Zulauf gewährleistet, ohne dass die Wirtschaftlichkeit der Anaerobprozesse beeinträchtigt wird.

In der Vorrichtung der Erfindung ist direkt am Zulauf des frischen Substrats zum Fermenter eine Apparatur eingebaut, in der
- frisches Substrat und Fermenterinhalt gezielt miteinander vermischt werden,
- -durch pneumatische Umwälzung wie Einblasen/Einpressen von Gas, wobei
- schwere Bestandteile wie Sand, Split, Steine nach unten sinken,
- die Sedimente gesammelt und
- die Sedimente gezielt und gesteuert ausgetragen werden.

Nachfolgend werden Ausführungsbeispiele der Erfindung beschrieben. Es zeigen
- Fig. 1 schematisch in Seiten- und Draufsicht eine erste Bauform mit trichterförmiger Apparatur, und
- Fig. 2 schematisch in Seiten- und Draufsicht eine zweite Bauform mit rohr- oder halbrohrförmiger Apparatur.

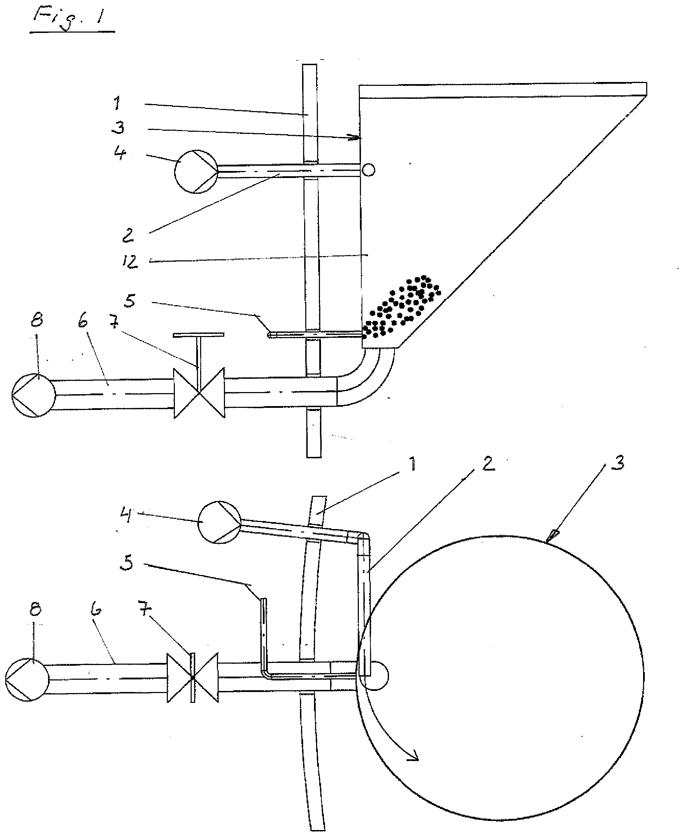

In Figur 1 ist vom Fermenter die Behälteraußenwand 1 angedeutet, durch die der Zulauf 2 für Ausgangssubstrat in eine Apparatur 3 im Innern des Fermenters geführt ist. Diese Apparatur 3 hat im Vertikalschnitt die Form eines Trichters 12, der nach oben offen ist und somit in kommunizierender Verbindung mit dem Fermenterinhalt steht, in den die Apparatur 3 eingetaucht ist.

Der Zulauf 2, der zweckmäßig mit einer Substratpumpe 4 bestückt ist, ist im oberen Drittel tangential in die Apparatur 3 geführt, so dass im Trichter 12 eine rotierende Strömung und damit eine Verwirbelung frischen Ausgangssubstrats mit oben durch die Trichteröffnung eintretendem Fermenterinhalt erreicht wird. Eine weitere Verbesserung der Verwirbelung wird erreicht durch Gaseinpressung mittels einer Leitung 5, die durch die Behälterwand 1 in den unteren Bereich der Apparatur 3 mündet.

Sedimente setzen sich am Boden des Trichters 12 ab und werden von dort über ein Rohr 6, zweckmäßig mit einem Absperrschieber 7 und einer Sandfangpumpe 8 bestückt, nach außerhalb des Fermenters ausgetragen.

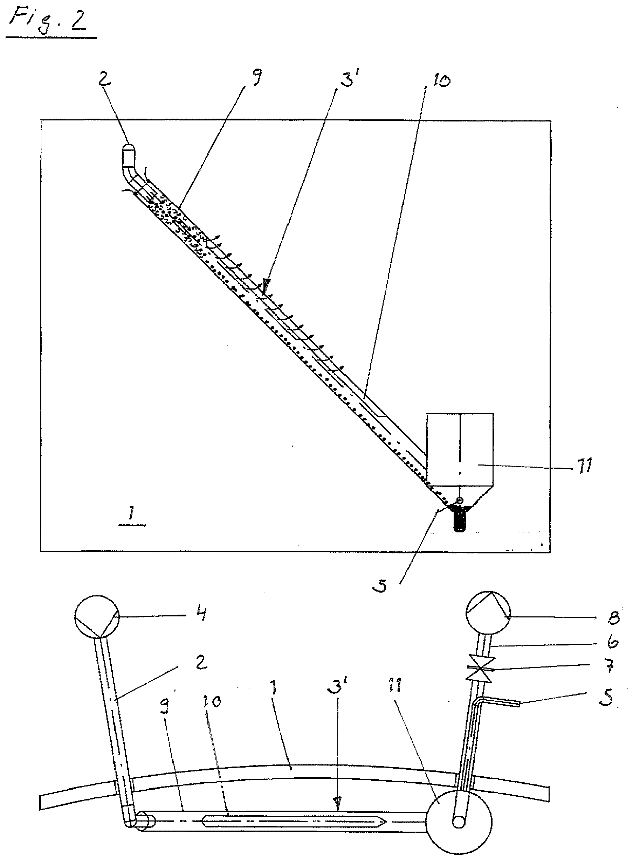

In Figur 2 ist vom Fermenter wiederum nur die Behälteraußenwand 1 angedeutet, durch die der Zulauf 2 für Ausgangssubstrat in eine Apparatur 3' im Innern des Fermenters geführt ist. Diese Apparatur 3' ist als ein nach unten geneigtes Mantelrohr 9 ausgebildet, in das oben der Zulauf 2 zentrisch einmündet. Das Mantelrohr 9 steht in kommunizierender Verbindung mit dem Fermenterinhalt, in den es eingetaucht ist.

Durch das einströmende frische Ausgangssubstrat wird eine Injektorströmung erzeugt, die Fermenterinhalt in das Mantelrohr 9 zieht und mit dem frischen Substrat vermischt. Der Rohrquerschnitt ist in einem mittleren Abschnitt 10 nach oben geöffnet, so dass weitere Vermischung mit Fermenterinhalt stattfinden kann.

Der Rohrapparat ist so weit geneigt, dass Sedimente durch Schwerkraft nach unten gleiten können. Am unteren Ende des Mantelrohrs 9 ist der Querschnitt wieder geschlossen und mit einem nach oben offenen Behälter 11 bestückt. Eine weitere Verbesserung der Verwirbelung wird erreicht durch Gaseinpressung mittels einer Leitung 5, die durch die Behälterwand 1 in den unteren Bereich des Behälters 11 mündet. Sedimente setzen sich am unteren Ende des Mantelrohrs 9 bzw. am Boden des Behälters 11 ab und werden von dort über ein Rohr 6, zweckmäßig mit einem Absperrschieber 7 und einer Sandfangpumpe 8 bestückt, nach außerhalb des Fermenters ausgetragen.

## Patentansprüche

1. Verfahren zur Abscheidung schwerer Bestandteile aus hochviskosem Ausgangssubstrat von Biogasanlagen im Innern eines Fermenters durch Verdünnen des Substrats zur Verminderung von dessen Viskosität und Abscheiden mitgeführter schwerer Bestandteile von Sand und/oder Split und/oder Steinen durch ihre Schwere,
**dadurch gekennzeichnet, dass**
eine Verdünnung frischen Ausgangssubstrats mit Fermenterinhalt erfolgt, dessen Viskosität durch Abbauprozesse im Fermenter reduziert ist, wobei das frische Ausgangssubstrat und der Fermenterinhalt durch Gaseinblasung miteinander vermischt werden.

2. Verfahren nach Anspruch 1,
**dadurch gekennzeichnet, dass** frisches Ausgangssubstrat und Fermenterinhalt durch hydraulische Strömung miteinander vermischt werden.

3. Verfahren nach Anspruch 1 oder 2,
**dadurch gekennzeichnet, dass** schwere Bestandteile wie Sand, Split, Steine gesammelt und ausgeschleust werden.

4. Vorrichtung zur Abscheidung schwerer Bestandteile von Sand und/oder Split und/oder Steinen aus hochviskosem Ausgangssubstrat in einer Biogasanlage mit einem Fermenter,
**dadurch gekennzeichnet, dass** am Zulauf (2) frischen Ausgangssubstrats zum Fermenter eine Apparatur (3,3') im Innern des Fermenters angeordnet ist,
a) zur Vermischung des Ausgangssubstrats mit Fermenterinhalt, der eine durch Abbauprozesse reduzierte Viskosität besitzt, und
b) zum Austragen angesammelter schwerer Bestandteile, wobei die Apparatur (3, 3') zur Vermischung des frischen Ausgangssubstrats mit dem Fermenterinhalt Einrichtungen (5) zum Einblasen von Gas besitzt.

5. Vorrichtung nach Anspruch 4,
**dadurch gekennzeichnet, dass** die Apparatur (3, 3') zur Vermischung frischen Ausgangssubstrats mit Fermenterinhalt Einrichtungen für eine hydraulische oder pneumatische Umwälzung besitzt.

6. Vorrichtung nach Anspruch 4 oder 5,
**dadurch gekennzeichnet, dass** die Apparatur (3) zur Vermischung frischen Ausgangssubstrats mit Fermenterinhalt in Form eines Trichters (12) ausgebildet ist, der in kommunizierender Verbindung mit dem Fermenterinhalt steht, und dass der Zulauf (2) für frisches Ausgangssubstrat tangential in den Trichter (12) geführt ist.

7. Vorrichtung nach Anspruch 4 oder 5,
**dadurch gekennzeichnet, dass** die Apparatur (3') zur Vermischung frischen Ausgangssubstrats mit Fermenterinhalt in Form eines nach unten geneigten Mantelrohres (9) ausgebildet ist, das in kommunizierender Verbindung mit dem Fermenterinhalt steht, und dass der Zulauf (2) für frisches Ausgangssubstrat zentrisch in dieses Mantelrohr (9) mündet.

8. Vorrichtung nach einem der Ansprüche 4 - 7,
**dadurch gekennzeichnet, dass** die Apparatur (3,3') zur Vermischung frischen Ausgangssubstrats mit Fermenterinhalt im Innern des Fermenters angeordnet und in den Fermenterinhalt eingetaucht ist.

9. Vorrichtung nach Anspruch 8 mit Anspruch 6,
**dadurch gekennzeichnet, dass** der Trichter (12) oben offen ist und unten mit einem Rohr (6) zum Abzug des Sediments nach außerhalb des Fermenters ausgebildet ist.

10. Vorrichtung nach Anspruch 8 mit Anspruch 7,
**dadurch gekennzeichnet, dass** das Mantelrohr (9) im weiteren Verlauf nach oben geöffnet und am unteren Ende wieder geschlossen und zum Abzug des Sediments nach außerhalb des Fermenters geführt ist.

11. Vorrichtung nach Anspruch 10,
**dadurch gekennzeichnet, dass** das Mantelrohr (9) am unteren Ende in einen nach oben offenen Behälter (11) mündet, von dem unten ein Rohr (6) zum Abzug des Sediments nach außerhalb des Fermenters führt.

## Claims

1. Method for separating heavy components from a high-viscosity starting substrate of biogas plants in the interior of a fermenter by diluting the substrate to reduce the viscosity thereof and separating entrained heavy components of sand and/or split and/or stones by weight,
**characterised in that**
a dilution of fresh starting substrate takes place with fermenter content of which the viscosity has been reduced by decomposition processes in the fermenter, the fresh starting substrate and the fermenter content being mixed together by blowing in gas.

2. Method according to claim 1,
**characterised in that** fresh starting substrate and fermenter content are mixed together by hydraulic flow.

3. Method according to either claim 1 or claim 2,
**characterised in that** heavy components such as sand, split, stones are collected and discharged.

4. Device for separating heavy components of sand and/or split and/or stones from a high-viscosity starting substrate in a biogas plant comprising a fermenter,
**characterised in that**, at the feed line (2) of fresh starting substrate to the fermenter, an apparatus (3, 3') is arranged in the interior of the fermenter
a) for mixing the starting substrate with fermenter content which has a viscosity reduced by decomposition processes and
b) for removing accumulated heavy components, the apparatus (3, 3') having devices (5) for blowing in gas to mix the fresh starting substrate with the fermenter content.

5. Device according to claim 4,
**characterised in that** the apparatus (3, 3') has devices for hydraulic or pneumatic circulation for mixing fresh starting substrate with fermenter content.

6. Device according to either claim 4 or claim 5, **characterised in that** the apparatus (3) is formed for mixing fresh starting substrate with fermenter content in the form of a funnel (12), which is in a communicative connection with the fermenter content, and **in that** the feed line (2) for fresh starting substrate passes into the funnel (12) tangentially.

7. Device according to either claim 4 or claim 5,
**characterised in that** the apparatus (3') for mixing fresh starting substrate with fermenter content is formed as a downwardly inclined casing pipe (9), which is in a communicative connection with the fermenter content, and **in that** the feed line (2) for fresh starting substrate opens into this casing pipe (9) centrally.

8. Device according to any of claims 4 - 7,
**characterised in that** the apparatus (3, 3') for mixing fresh starting substrate with fermenter content is arranged in the interior of the fermenter and immersed in the fermenter content.

9. Device according to claim 8 in conjunction with claim 6,
**characterised in that** the funnel (12) is formed open upwards and with a pipe (6) downwards to extract the sediment to outside the fermenter.

10. Device according to claim 8 in conjunction with claim 7,
**characterised in that** the casing pipe (9) is open in the continued progression upwards and closed again at the lower end and passes outside the fermenter to extract the sediment.

11. Device according to claim 10,
**characterised in that** the casing pipe (9) opens, at the lower end, into an upwardly open container (11), from which a pipe (6) for extracting the sediment passes outside the fermenter.

## Revendications

1. Procédé de séparation de composants lourds d'un substrat de départ à haute viscosité d'installations de biogaz à l'intérieur d'un fermenteur par dilution du substrat pour diminuer sa viscosité et séparer des constituants lourds entraînés à base de sable, et/ou de gravier, et/ou de pierres, du fait de leur poids
**caractérisé en ce**
**qu'**une dilution de substrat de départ frais a lieu avec le contenu du fermenteur dont la viscosité est réduite par des processus de dégradation, dans lequel le substrat de départ frais et le contenu du fermenteur sont mélangés l'un à l'autre par une incorporation de gaz par soufflage.

2. Procédé selon la revendication 1,
**caractérisé en ce que** du substrat de départ frais et le contenu du fermenteur sont mélangés l'un à l'autre par un écoulement hydraulique.

3. Procédé selon la revendication 1 ou la revendication 2,
**caractérisé en ce que** des constituants lourds, comme du sable, du gravier, des pierres, sont rassemblés et sont évacués.

4. Dispositif destiné à la séparation de composants lourds à base de sable, et/ou de graviers et/ou de pierres, d'un substrat de départ à haute viscosité dans une installation de biogaz à l'aide d'un fermenteur,
**caractérisé en ce qu'**au niveau de la conduite d'apport (2) de substrat de départ frais dans le fermenteur, un appareillage (3,3') est disposé à l'intérieur du fermenteur,
a) pour le mélange du substrat de départ avec le contenu du fermenteur, qui possède une viscosité réduite par les processus de dégradation, et
b) pour l'évacuation de constituants lourds rassemblés, l'appareillage (3, 3') possédant des dispositifs (5) pour l'incorporation de gaz par soufflage afin de mélanger le substrat de départ frais avec le contenu du fermenteur.

5. Dispositif selon la revendication 4,
**caractérisé en ce que** l'appareillage (3, 3') destiné au mélange de substrat de départ frais avec le contenu du fermenteur possède des dispositifs pour assurer une circulation hydraulique ou pneumatique.

6. Dispositif selon la revendication 4 ou la revendication 5,
**caractérisé en ce que** l'appareillage (3) destiné au mélange de substrat de départ frais avec le contenu du fermenteur est conçu sous la forme d'un entonnoir (12) qui est en liaison communicante avec le contenu du fermenteur et que la conduite d'apport (2) pour du substrat de départ frais est menée de manière tangentielle dans l'entonnoir (12).

7. Dispositif selon la revendication 4 ou la revendication 5,
**caractérisé en ce que** l'appareillage (3') pour le mélange de substrat de départ frais avec le contenu du fermenteur est conçu sous la forme d'un tube enveloppe (9) incliné vers le bas qui est en liaison communicante avec le contenu du fermenteur et que la conduite d'apport (2) pour du substrat de départ frais débouche au centre dans ce tube enveloppe (9).

8. Dispositif selon l'une des revendications 4 à 7,
**caractérisé en ce que** l'appareillage (3,3') pour le mélange de substrat de départ frais avec le contenu du fermenteur est disposé à l'intérieur du fermenteur et est immergé dans le contenu du fermenteur.

9. Dispositif selon la revendication 8 conjointement à la revendication 6,
**caractérisé en ce que** l'entonnoir (12) est ouvert vers le haut et est conçu en bas avec un tuyau (6) pour l'évacuation du sédiment vers l'extérieur du fermenteur.

10. Dispositif selon la revendication 8, conjointement à la revendication 7,
**caractérisé en ce que** le tube enveloppe (9) dans l'extension supplémentaire est ouvert vers le haut et est de nouveau fermé à l'extrémité inférieure et mène vers l'extérieur du fermenteur pour l'évacuation du sédiment.

11. Dispositif selon la revendication 10,
**caractérisé en ce que** le tube enveloppe (9) débouche au niveau de l'extrémité inférieure dans un récipient (11) ouvert vers le haut à partir duquel, en dessous, un tuyau (6) mène vers l'extérieur du fermenteur pour l'évacuation du sédiment.
